# EUROPEAN PATENT APPLICATION

(11) **EP 4 641 023 A1**
(43) Date of publication of application: **29.10.2025**
(21) Application number: 25171523.1
(22) Date of filing: 21.04.2025
(51) Int. Cl.: F04D 25/10, F04D 29/70, F24F 6/12, F04D 29/60

(54) **LIFTING SHAFT FAN**

(30) Priority: 23.04.2024 TW 113204036 U
(71) Applicant: Sui, Cheng-Chung, 434 Taichung City (TW)
(72) Inventor: Sui, Cheng-Chung, 434 Taichung City (TW)
(74) Representative: Wittmann, Günther

(57) **Abstract**

A lifting shaft fan includes a fan, a rotator, a Near-filed communication component, a platform, an atomizer, a lifting shaft, a foundation, at least two wheels and a power supply. The fan configures on the rotator. The rotator connects to the Near-filed communication component, and the rotator configures on the platform. The lifting shaft configures under the platform and configures on the foundation. The at least two wheels configures under the foundation. The power supply supplies power to the rotator, Near-filed communication component, the atomizer and the lifting shaft. Controlling the Near-filed communication component via mobile APP. The Near-filed communication component controls the rotator. The rotator drives the fan to rotate. Using the lifting shaft to adjust the overall height of the lifting shaft fan. In particular, the atomizer configures above the platform, and the mist which is generated by the atomizer has disinfection function.

## Description

### TECHNICAL FIELD

The present invention relates to an electrical device, especially for a lifting shaft fan that may be controlled by mobile APP.

### BACKGROUND OF RELATED ARTS

The height of the household electric fan that can be adjusted is limited. Furthermore, the foundation of household electric fan is fixed foundation. If the user wants to move the electric fan, he/she can only manually lift the entire electric fan and move the electric fan to a specific location. The afore-mentioned manual way is inconvenient for use sometimes. Currently, most of the electric fans are used to purify the air, but fewer electric fans can reduce bacteria and virus in the air or the environment.

### SUMMARY

To solve the problems of well-known technology, the purpose of the present invention is to provide a lifting shaft fan that may be controlled by mobile APP and may perform environment disinfection. The APP may be a software and/or a computer program product that runs on a processor of a computer having a memory.

The lifting shaft fan of the present invention includes a fan, a rotator, a Near-filed communication component, a platform, an atomizer, a lifting shaft, a foundation, at least two wheels and a power supply. In one embodiment, the Near-filed communication component may be a Bluetooth^{™} component or the like.

In the present invention, the fan configures on the rotator. The rotator connects to the Near-filed communication component, and the rotator configures above the platform. The lifting shaft configures under the platform but configures above the foundation. The at least two wheels configure under the foundation. The power supply supplies power to the rotator, the Near-filed communication component, the atomizer and the lifting shaft.

The technical function of the present invention is controlling the Near-filed communication component via mobile APP. The Near-filed communication component controls the rotator. The rotator actuates the fan to rotate. The lifting shaft is used to adjust the overall height of the lifting shaft fan. In particular, the mist which is generated by the atomizer has disinfection function.

### BRIEF DESCRIPTION OF THE DRAWINGS

Other features and functions of the present invention will be clearly presented in the embodiments shown in the accompanying drawings.
FIG. 1 shows a solid diagram of the lifting shaft fan of an embodiment of the present invention.
FIG. 2 shows a side view diagram of the lifting shaft fan of the embodiment of the present invention.
FIG. 3 shows a side view diagram of the lifting shaft fan of the embodiment of the present invention.

### DETAILED DESCRIPTION OF THE INVENTION

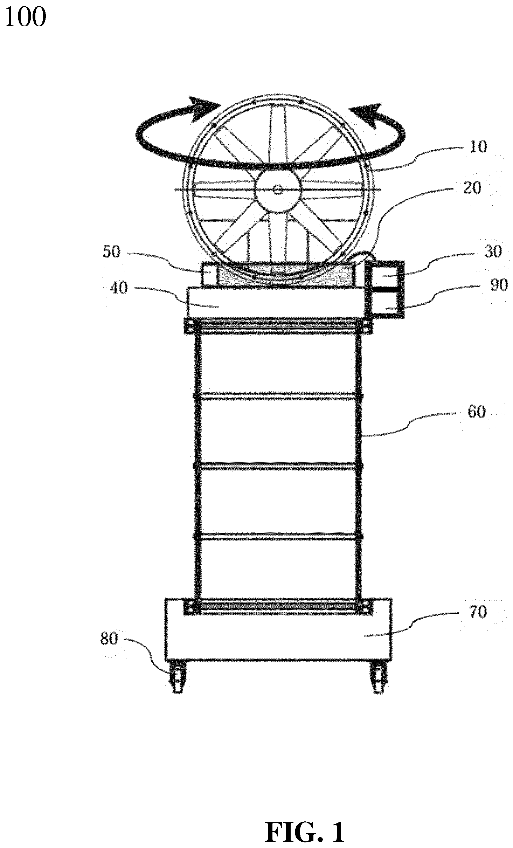

Please refer to FIG. 1, the invention provides a lifting shaft fan 100 which is illustrated in the present embodiment includes a fan 10. The fan 10 connects to a rotator 20 and the fan 10 is configured above a platform 40. The rotator 20 is controlled by a Near-filed communication component 30 to actuate the rotation of the fan 10. The lifting shaft 60 which connects to bottom of the platform 40 controls the entire height of the platform 40. The other end of the lifting shaft 60 is configured on a foundation 70, and at least two wheels 80 connects to the bottom of the foundation 70 to make the lifting shaft fan 10 movable. The power supply 90 supplies power to the rotator 20, the Near-filed communication component 30, the atomizer 50 and the lifting shaft 60. In one embodiment, the Near-filed communication component may be a Bluetooth^{™} component or the like.

Specifically, the fan 10 connects to the rotator 20, and the rotator20 connects to the Near-filed communication component 30. The power supply 90 supplies power to the rotator 20 and the Near-filed communication component 30 which is configured on the platform 40. The fan 10 is axial fan. The fan 10 is propeller fan, tube axial fan or blade wheel fan. The rotator 20 may make the fan 10 oscillate. The oscillation means that the fan 10 may be rotated from left to right, and the wind which is generated by the fan 10 may be delivered in different directions. Specifically, the angle range of oscillation of the fan 10 is ranged from 0 degree to 300 degrees. Furthermore, the fan 10 may also be fixed without oscillation, and the fan 10 may blow wind which is generated by the fan 10 to certain direction. The Near-filed communication component 30 of the present embodiment is used to control the operation mode of the rotator 20, and thus the fan 10 may be operated in different modes. In detail, the rotator 20 is a motor. The motor is alternating current motor or direct current motor.

The Near-filed communication component 30 is Near-filed communication motor variable-frequency controller. The Near-filed communication motor variable-frequency controller may receive the Near-filed communication signal. Therefore, the mobile APP may be used to send Near-filed communication signal to the Near-filed communication motor variable-frequency controller. Specifically, the Near-filed communication motor variable-frequency controller is Bluetooth^{™} motor variable-frequency controller. When the Near-filed communication motor variable-frequency controller receives the Near-filed communication signal, the Near-filed communication motor variable-frequency controller outputs the Near-filed communication signal to the rotator 20, and the rotator 20 actuates the fan 10 to operate. Specifically, please refer to FIG. 1. The mobile APP sends Near-filed communication signal to make the fan 10 oscillate. After the Near-filed communication motor variable-frequency controller receives the Near-filed communication signal which is going to make the fan 10 oscillate, the Near-filed communication motor variable-frequency controller outputs the oscillating signal of the fan 10 to the rotator 20, and the rotator 20 makes the fan 10 be operated in an oscillating mode. In particular, the Near-filed communication component 30 of the present invention is a Near-filed communication motor variable-frequency controller. The Near-filed communication motor variable-frequency controller may automatically change the frequency and output different frequencies to a motor of the fan 10, and let the motor of the fan 10 perform different speed to adjust the wind speed of the fan 10 and adjust the air delivery which is generated by the fan 10. The motor of the fan 10 is alternating current motor or direct current motor. The Near-filed communication motor variable-frequency controller of the present invention may let the fan 10 oscillate or let the fan 10 fix in one direction, and the Near-filed communication motor variable-frequency controller further adjusts the wind speed of the fan 10. The APP may be a software and/or a computer program product that runs on a processor of a computer having a memory.

Further, the Near-filed communication component 30 connects to the power supply 90. User uses the mobile APP to output the Near-filed communication signal to the Near-filed communication motor variable-frequency controller. The Near-filed communication motor variable-frequency controller outputs signal to the power supply 90 to control the power supply 90 for turning it on or turning it off. Specifically, user may use the mobile APP to output the Near-filed communication signal which turns the power supply 90 on the Near-filed communication motor variable-frequency controller. The Near-filed communication motor variable-frequency controller thereafter outputs signal to the power supply 90 and turns the power supply 90 on. After power supply 90 receives the Near-filed communication signal, the power supply 90 turns the power source on and supplies power to the lifting shaft fan 100, therefore to let the lifting shaft fan 100 operate normally. Especially for convenient to use, user may use the mobile APP to output the Near-filed communication signal which contains automatic shutdown time to the Near-filed communication motor variable-frequency controller. When the set shutdown time is reached, the Near-filed communication motor variable-frequency controller outputs signal and turns the power supply 90 off. However, for safety reasons, user is forbidden use mobile APP for sending automatic starting time signal.

In particular, the atomizer 50 configures above the platform 40 and behind the fan 10. The power supply 90 supplies power to the atomizer 50, and let the atomizer 50 generate mist. The atomizer 50 of the present embodiment is a hypochlorous acid water atomizer, but the present embodiment is not limited thereto. When the fan 10 is operating, the fan 10 collects in and blows the mist which is generated by the hypochlorous acid water atomizer that is behind the fan 10. Furthermore, the hypochlorous acid water atomizer connects to a the hypochlorous acid water refill bucket to continuously supply the hypochlorous acid water to the hypochlorous acid water atomizer, and thus the hypochlorous acid water atomizer may continuously generate mist. Specifically, a hypochlorous acid water storage container of the hypochlorous acid water atomizer is equipped with an automatic liquid level control component to maintain the hypochlorous acid water atomizer at an optimal mist generation rate.

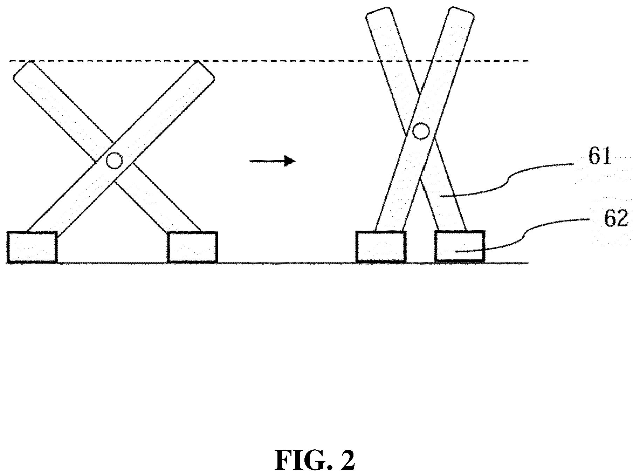

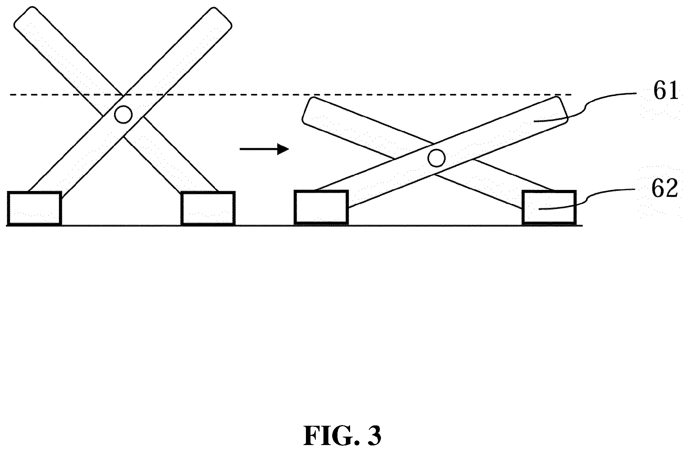

Please refer to FIG. 1, the fan 10 connects to the rotator 20, and the fan 10 configures above the platform 40. The lifting shaft 60 configures below the platform 40. The lifting shaft 60 is a vertical lifting shaft that may be lifted vertically from the reference ground. When the lifting shaft 60 is lifted vertically, the height of the platform 40 may be adjusted to make the fan 10 close to the ground or away from the ground. The lifting shaft 60 is a Z axis lifting shaft in the present embodiment. The Z axis lifting shaft is constructed by a hand wheel, plurality X type lifter 61 and plurality push rod. User manually rotates the hand wheel to adjust the Z axis lifting shaft. Specifically, please refer to FIG. 2. When the hand wheel is manually rotated in a clockwise direction, the push rod 62 provides resistance to two ends of X type lifter 61. The two ends of the X type lifter 61 are getting closer to each other via the reference point such as the center point of X type lifter 61, thereby increasing the height of the X type lifer 61 and raising the platform 40. Further, please refer to FIG. 3. When the hand wheel is manually rotated in a counterclockwise direction, the push rod 62 provides space of the two ends of the X type lifter 61. The two ends of the X type lifter 61 may move in opposite directions based on the center point of the X type lifter 61, thereby reducing the height of the entire X type lifter 61 and making the platform 40 to descend. The height adjustment range of Z axis lifting shaft is ranged from 0 meter to 1 meter. The highest height of the lifting shaft fan 100 from ground to top of the fan 10 is 1.5 meter. On the other hand, the lowest height of the lifting shaft fan 100 from ground to top of the fan 10 is 0.5 meter.

The other end of the lifting shaft 60 configures above a foundation 70, and the at least two wheels 80 configure below the foundation 70. The number of at least two wheels are 2 to 4. The at least two wheels 80 of the present embodiment are four wheels, and the four wheels are caster wheels. Therefore, the lifting shaft fan 100 may be freely moved. After the lifting shaft fan 100 is moved to a specific location, user may lock rotation of the caster wheels and make caster wheels fail to move. The lifting shaft fan 100 may be fixed and the body per se may be stabilized in a specific location.

In summary, the lifting shaft fan 100 of the present invention is suitable for controlling the operation of the fan 10 with the Near-filed communication component 30, and user uses mobile APP to send Near-filed communication signal to Near-filed communication motor variable-frequency controller. After the Near-filed communication motor variable-frequency controller receives the Near-filed communication signal, the Near-filed communication motor variable-frequency controller outputs signal to the rotator 20 to control the fan 10 to oscillate or not during operation. User may use the mobile APP to send the Near-filed communication signal to the Near-filed communication motor variable-frequency controller, and the Near-filed communication motor variable-frequency controller outputs a signal to the motor of the fan 10 to control the wind speed of the fan 10 after receiving Near-filed communication signal. User may use the mobile APP to send the Near-filed communication signal to the Near-filed communication motor variable-frequency controller and the Near-filed communication motor variable-frequency controller outputs a signal to the power supply 90 to control the ON/OFF or the automatic starting/shutdown time functions of the power supply 90 after the Near-filed communication motor variable-frequency controller receives the Near-filed communication signal. Furthermore, the lifting shaft 60 can lift the platform 40 to adjust the height of the fan 10 which configures above the platform 40, and the foundation 70 has at least two wheels 80 that may make the lifting shaft fan 100 freely move. In particular, the mist which is generated by the hypochlorous acid water atomizer may disinfect the air, and the lifting shaft fan 100 may indeed achieve the purpose of the present invention.

As is understood by a person skilled in the art, the foregoing preferred embodiments of the present invention are illustrated of the present invention rather than limiting of the present invention. It is intended to cover various modifications and similar arrangements included within the spirit and scope of the appended claims, the scope of which should be accorded the broadest interpretation so as to encompass all such modifications and similar structure. While the preferred embodiment of the invention has been illustrated and described, it will be appreciated that various changes can be made therein without departing from the spirit and scope of the invention.

## Claims

1. A lifting shaft fan, comprising:
a fan;
a rotator, connected to the fan;
a platform, connected to the rotator;
a Near-filed communication component, connected to the rotator;
an atomizer, configured above the platform;
a lifting shaft, connected under the platform;
a foundation, connected under the lifting shaft;
at least two wheels, connected under the foundation; and
a power supply, connected to the rotator, the Near-filed communication component, the atomizer and the lifting shaft respectively.

2. The lifting shaft fan as claimed in claim 1, wherein the fan is axial fan.

3. The lifting shaft fan as claimed in claim 1, wherein the fan is propeller fan, tube axial fan or blade wheel fan.

4. The lifting shaft fan as claimed in claim 1, wherein the rotator is motor.

5. The lifting shaft fan as claimed in claim 4, wherein the motor is alternating current motor or direct current motor.

6. The lifting shaft fan as claimed in claim 1, wherein the Near-filed communication component is Near-filed communication motor variable-frequency controller.

7. The lifting shaft fan as claimed in claim 1, wherein the lifting shaft is vertical lifting shaft.

8. The lifting shaft fan as claimed in claim 1, wherein the lifting shaft is horizontal Z axis lifting shaft.

9. The lifting shaft fan as claimed in claim 1, wherein numbers of the at least two wheels are 2 to 4.

10. The lifting shaft fan as claimed in claim 1, wherein the at least two wheels are caster wheels.
